# EUROPEAN PATENT SPECIFICATION

(11) **EP 4 153 084 B1**
(45) Date of publication and mention of the grant of the patent: **09.07.2025**
(21) Application number: 21721573.0
(22) Date of filing: 03.05.2021
(51) Int. Cl.: A61B 34/20, G06N 3/02, G06N 20/00, A61B 90/00, A61B 34/10

(54) **APPARATUS FOR DETECTING AND TRACKING THE POSITION AND/OR DEFORMATION OF A BODY ORGAN**
VORRICHTUNG ZUR ERFASSUNG UND VERFOLGUNG DER POSITION UND/ODER VERFORMUNG EINES KÖRPERORGANS
APPAREIL DE DÉTECTION ET DE SUIVI DE LA POSITION ET/OU DE LA DÉFORMATION D'UN ORGANE CORPOREL

(30) Priority: 25.06.2020 IT 202000015322
(43) Date of publication of application: 29.03.2023
(73) Proprietor: IO Surgical Research S.r.l., 16145 Genova (GE) (IT)
(72) Inventor: MASTROGIOVANNI, Fulvio, 16147 Genova (IT); TERRONE, Carlo, 10123 Torino (IT); TRAVERSO, Paolo, 16145 Genova (IT)
(74) Representative: Brunacci, Marco
(86) International application number: PCT/EP2021/061517
(87) International publication number: WO 2021/259537

(56) References cited:
- WO-A1-2012/006636
- WO-A1-2017/151904
- DAN WANG ET AL: "Real Time 3D Visualization of Intraoperative Organ Deformations Using Structured Dictionary", IEEE TRANSACTIONS ON MEDICAL IMAGING, IEEE SERVICE CENTER, PISCATAWAY, NJ, US, vol. 31, no. 4, 1 April 2012 (2012-04-01), pages 924 - 937, XP011491076, ISSN: 0278-0062, DOI: 10.1109/TMI.2011.2177470

## Description

The present invention relates to an apparatus and a method for detecting and tracking the position and deformation of a body organ in an intraoperative context, for example during surgical procedures.

The known systems presently being developed to achieve an assisted intracorporeal orientation aided by augmented reality techniques in the course of surgical procedures in an intraoperative context are based on a reconstruction of the three-dimensional augmented reality image of a body organ, which can be obtained using various techniques.

Based on the availability of such three-dimensional augmented reality images, there exist different known approaches that enable an association of the image with the organ to be obtained, and the tracking thereof in order to allow an assisted intracorporeal orientation in the course of surgical procedures in that intraoperative context.

Such traditional approaches can for example regard the superimposition of that image in the common space and manual tracking in the visual field of medical staff, for example a surgeon, by means of known augmented reality techniques.

Such approaches, and others based on similar technologies, are usable in an intraoperative context, for example during a surgical procedure, but they do not enable a valid, effective, certifiable real-time synchrony with the visual field of medical staff, for example a surgeon, by means of augmented reality techniques, both as regards an unsatisfactory precision in determining the position and/or deformation information and/or variations associated with that position and/or deformation of the organ, and as regards the reproduction thereof in the three-dimensional augmented reality image.

Possible systems known in the prior art that could be adopted for the purpose of detecting the position and/or deformation and/or variations associated with that position and/or deformation of an organ in a satisfactory manner, relate to body imaging techniques for diagnostic use, such as, for example, X-rays, computed tomography (CT), magnetic resonance imaging (MRI), and techniques based on similar principles.

These techniques are not utilisable in an intraoperative context, for example during a surgical operation, because their results are as a rule available offline, and can thus not be used to update the position and/or deformation related to the three-dimensional augmented reality image in correspondence with that position and/or deformation and/or variations associated with that position and/or deformation of the organ of which the augmented reality image is a representation.

Moreover, such techniques require equipment that is not compatible with an intraoperative context, especially in the course of a surgical operation.

At the current state of the art, the most common technologies that could be adopted in an intraoperative context, for example during a surgical operation, and which are not based on approaches related to body imaging for diagnostic use, are the following, or in any case they are based on similar principles:
- Cameras, or devices of a similar nature, arranged in the room and/or fixed in an appropriate and advantageous manner within the room near relevant equipment. Such cameras or devices of a similar nature can be used to frame the intraoperative context, for example during a surgical operation, and provide visual information from which to extract, by means of algorithms of an inductive or deductive type, characteristics useful for determining the position and/or deformation and/or variations associated with that position and/or deformation of the body organ.
- Motion capture systems, or devices of a similar nature, based on cameras and fiducial markers, in which the cameras are positioned in the room and/or fixed in an appropriate and advantageous manner within the room near relevant equipment, and in which the fiducial markers are mechanically fixed in an appropriate and advantageous manner to the body organ subject to the intraoperative context, for example during a surgical procedure.

Such motion capture systems, or devices of a similar nature, can be used to detect the position and/or the variations associated with the position of one or more fiducial markers mechanically coupled to the organ, and thus provide position information about the fiducial markers from which to extract, by means of algorithms of an inductive or deductive type, characteristics useful for determining the position and/or deformation and/or variations associated with that position and/or deformation of the body organ to which the fiducial markers are fixed.
- Location systems based on radio signals, such as, for example, the ones based on Wi-Fi technology or the like, in which at least one Wi-Fi signal emitter is positioned in the room and/or fixed in an appropriate and advantageous manner within the room near relevant equipment, and in which modulation data of that signal, such as, for example, amplitude and phase, can be considered information attributable, by means of algorithms of an inductive or deductive type, to characteristics useful for determining the position and/or deformation and/or variations associated with that position and/or deformation of the body organ.

These technologies and those based on similar principles are often difficult to use in an intraoperative context, for example in the course of a surgical procedure, where camera-based systems might not frame the intraoperative context in an adequate manner and/or with the necessary precision, motion capture systems might not detect a sufficient number of fiducial markers mechanically coupled to the body organ in an adequate manner, and location systems based on radio signals might not be capable of isolating that organ in order to determine the position and/or deformation and/or variations associated with that position and/or deformation, and/or in any case in a sufficiently precise and reliable manner to determine a representation of the position and/or deformation of the organ in the course of that intraoperative context, especially during a surgical procedure.

These technologies and those based on similar principles often do not ensure satisfactory performances in terms of precision of the determination of the position and/or deformation and/or variations associated with that position and/or deformation of a body organ in an intraoperative context, for example during a surgical operation, a context that is not suitable for the determination of the position and/or deformation using, for example:
- information obtained from cameras, because of occlusions due to the particular configuration of the intraoperative context, such as, for example, the presence of objects, medical devices, medical staff or other body organs, which are interposed between the cameras and the body organ whose position and/or deformation it is desired to determine, or because of the non-appropriate resolution of that information for the purpose of determining that position and/or deformation, due to the distance at which the cameras must be placed in order not to negatively impact the intraoperative context, such as, for example, the need not to disturb the operations of medical staff, for example a surgeon, who is working there;
- information obtained from motion capture systems, because of occlusions due to the particular configuration of the intraoperative context, such as, for example, the presence of medical staff, objects, medical devices, or other body organs, which are interposed between the cameras and one or more fiducial markers mechanically coupled to the body organ whose position and/or deformation it is desired to determine, or because of the need to simultaneously detect a sufficient number of such fiducial markers in order to determine that position and/or deformation, due to positions often not suited to the purpose for which such fiducial markers can be placed on that organ so as not to negatively impact the intraoperative context, such as, for example, the need not to impede the freedom of the actions of medical staff, for example a surgeon, who is working there;
- information obtained from location systems based on radio signals, such as, for example, the ones based on Wi-Fi technology or the like, because of the inability of such signals to isolate and segment the body organ whose position and/or deformation it is desired to determine, or because of the inadequate precision with which such segmentation can be done, as a result of fluctuations of the modulations of the radio signals due to the environment in which the intraoperative context, for example a surgical procedure, arises.

A typical example of an unsuitable environment is represented by areas, zones, or rooms used for activities of a medical nature where various objects for medical use, various medical devices, and various members of the medical staff are present, such as, for example, operating rooms in which surgeons operate, or in general areas, zones, or rooms where an intraoperative context can arise.

Thus there is a felt need to improve the known systems and methods for detecting and tracking the position and deformation of a body organ in an intraoperative context, for example in the course of surgical procedures.

WO2012006636 discloses a detection apparatus according to the preamble of claim 1.

The technical task of the present invention is therefore to provide an apparatus for detecting and tracking the position and deformation of a body organ in an intraoperative context, for example in the course of surgical procedures, which allows the aforementioned technical drawbacks of the prior art to be eliminated.

Within the scope of this technical task one object of the invention is to provide an apparatus for detecting and tracking the position and deformation of a body organ in an intraoperative context, for example during surgical procedures, which enables a three-dimensional augmented reality image of that organ to be associated with said representation of the position thereof and/or deformation thereof, in a simple, effective and safe manner.

Another object of the invention is to provide an apparatus for detecting and tracking the position and deformation of a body organ in an intraoperative context, for example in the course of surgical procedures, which enables the assisted intracorporeal orientation to be guided in the course of surgical procedures in the intraoperative context.

Yet a further object of the invention is to provide an apparatus for detecting and tracking the position and deformation of a body organ in an intraoperative context, for example during surgical procedures, which provides data of an absolute or time-differential type localised on the surface of that organ.

The technical task, as well as these and other objects according to the present invention, are achieved by providing a detection and tracking apparatus for and tracking at least the position of a body organ subject to manipulation, according to claim 1.

In one embodiment of the invention, when the body organ under manipulation is rigid or almost rigid like the prostate , one single detection sensor can be enough.

In an embodiment of the invention, at least when the organ is deformable, at least two detection sensors for detecting the position and deformation of said body organ are provided for; said at least one transmitting unit being configured to transmit the information about said position and said deformation of an absolute or time-differential type detected by said detection sensors; said at least one receiving unit being configured to receive said information about said position and said deformation of an absolute or time-differential type transmitted by said transmitting unit said at least one processing unit being configured to determine a representation of said position and said deformation of said organ, associate a three-dimensional augmented reality image of said organ with said representation of said position and said deformation thereof, overlaying said three-dimensional augmented reality image with the position and deformation of said organ in the common three-dimensional space through said wearable and/or portable display device; tracking said position and said deformation of said three-dimensional augmented reality image in correspondence with the position and deformation of said organ of which said three-dimensional augmented reality image is a representation, comparing said information about said position and said deformation with a plurality of predefined models of position and deformation of organs.

The body organ is selected among thoracic internal organs, abdominal organs and pelvic organs.

In more detail, the body organ in selected among organs of the cardiovascular system comprising large arterial and venous vessels and heart, organs of the respiratory system comprising lungs and airway and mediastinal structures, organs of the digestive system comprising liver, esophagus, stomach, Gallbladder, pancreas, intestine, rectum intestine, splanchnic organs comprising spleen, and organs of the urinary and reproductive system comprising kidney, ureters, bladder, prostate, uterus, ovaries, vagina.

Other features of the invention are defined in the subsequent claims.

Additional features and advantages of the invention will become more apparent from the description of a preferred but not exclusive embodiment of an apparatus for detecting and tracking the position and/or deformation of a body organ which is subject to manipulation in an intraoperative context, for example in the course of surgical procedures according to the invention, illustrated by way of non-limiting example in the accompanying drawings, in which:

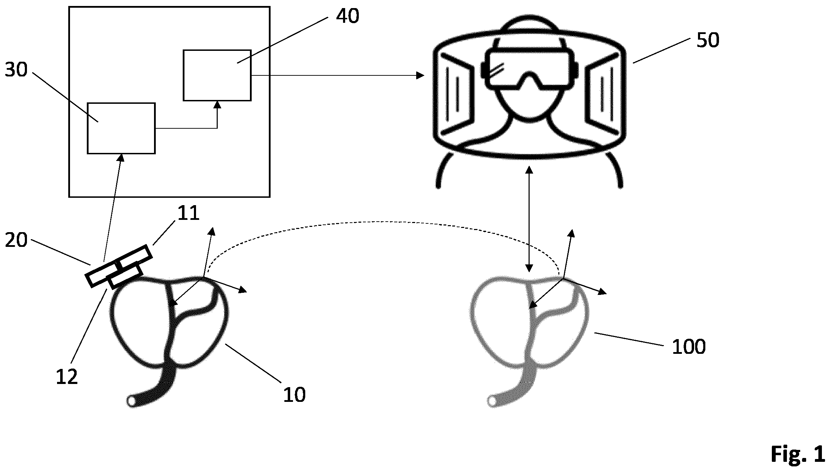

in figure 1 the invention is schematised and exemplified.

With reference to aforementioned figure, it shows an apparatus for detecting and tracking the position and/or deformation of a body organ which is subject to manipulation in an intraoperative context, for example in the course of surgical procedures, denoted in its entirety by the reference number (1).

The body organ (10) has at least one detection sensor (11) for detecting the position and/or deformation, configured to provide information of an absolute or time-differential type, and a rigid coupling means (12) for coupling the at least one sensor to the organ.

The sensor (11) detects information about the position and/or deformation and a transmitting unit (20) transmits the information to a receiving unit (30).

A processing unit (40), in communication with the receiving unit (30) and with at least one wearable and portable display device (50), is configured to perform successive steps of a procedure for evaluating the information detected by the detection sensor (11).

Through algorithms and procedures of an inductive or deductive type, the processing unit (40) determines a representation of the position and/or deformation of the body organ (10) in the course of the intraoperative context.

Said representation of the position and/or deformation of the body organ (10) can be in form of spatial coordinates of a set of points and angles, for instance spatial coordinates of a set of three points and three angles.

Then the processing unit (40) associates a three-dimensional augmented reality image (100) of the organ (10) with the representation of the position thereof and/or deformation thereof, and overlays the three-dimensional augmented reality image (100) on the body organ (10) in the common three-dimensional space through the wearable and/or portable display device (50), typically through known augmented reality techniques.

The processing unit (40) then tracks the position and/or deformation of the three-dimensional augmented reality image (100) in correspondence with the position and/or deformation of the body organ (10) of which the augmented reality image (100) is a representation in the course of the intraoperative context, comparing the information about the position and/or deformation and/or variations associated therewith with a plurality of predefined models of position and/or deformation and/or evolution of that position and/or deformation.

In an advantageous configuration of the apparatus, said parameters and said predefined models can be rendered specific for different types of internal body organs through a calibration step, whereby, by means of algorithms and procedures of a deductive type, it is possible, for every such type of internal body organ, to determine a model for determining the position and/or deformation and/or variations associated with that position and/or deformation.

Similarly, through a learning step based on algorithms and procedures of an inductive type, it is possible, for every type of internal body organ, to generalise a model for determining the position and/or deformation and/or variations associated with that position and/or deformation, as a consequence of actions of manipulation in an intraoperative context, for example in the course of surgical procedures.

Such actions correspond to conditions of usual behaviour in carrying out surgical procedures, there being available for this purpose an algorithm for predicting said position and/or deformation which is in the form of an inductive or deductive algorithm, such as, for example, a computational model based on a neural network or other approximation algorithms capable of carrying out learning cycles during the current use or procedures in a closed form.

By way of example, the inventors have been able to observe that, thanks to the use of algorithms of an inductive type, for example based on neural networks, it is possible to recognise and track the evolution of the position and/or deformation and/or variations associated with that position and/or deformation of an internal organ subject to an intraoperative context, such as, for example, a surgical procedure, by analysing solely differential movement data of that organ in that context, without the need to use cameras, motion capture systems, or location systems based on radio signals, as in the known systems.

All this with a clear benefit for the construction of a system of assisted intracorporeal orientation that is reliable in the course of that surgical procedure in that intraoperative context, and also in terms of the precision of the surgical procedure and cost reduction.

As a further example, the inventors have been able to observe that, thanks to the use solely of differential movement data, it is possible to associate a three-dimensional augmented reality image of the organ with said representation of the position thereof and/or deformation thereof, and then overlay the three-dimensional augmented reality image with the position and/or deformation of said organ in the common three-dimensional space through a wearable or portable display device, and then update the position and/or deformation of the three-dimensional augmented reality image in correspondence with the position and/or deformation of the organ, for example by means of augmented reality technologies, for the purpose of improving the performance of the surgical procedure.

The operation of the apparatus for detecting and tracking the position and/or deformation of a body organ subject to manipulation in an intraoperative context (1) according to the invention appears clear from what is described and illustrated and, in particular in a first preferred but not exclusive embodiment, it is substantially the following.

A three-dimensional scan of the body organ (10) is considered to be available, of which we define a particular instance as O, subjected to a surgical operation, to be used as a augmented reality image (100), of which we define a particular instance as I.

I is to be considered as a set RI of M elements, such that RI = (Q_1, ..., Q_i, ..., Q_M), where the generic element Q_i is a set of three values (x_i, y_i, z_i) which represents the position of Q_i in the common space with respect to a Cartesian reference system W, appropriately defined.

A display device (50) is considered to be available, of which we define a particular instance as a see-through display for augmented reality now identified as H, such as, for example, a commercial Microsoft Hololens or similar device or a 3D robotic visor.

N detection sensors (11) are considered to be available, typically inertial sensors, such as, for example, accelerometers, gyroscopes, magnetometers, devices identified as a particular instance of a set D_1, ..., D_i, ..., D_N, from which data can be acquired either via a cable or by means of a wireless connection.

The devices D_1, ..., D_i, ..., D_N can be secured to the organ O by means of a coupling means (12), typically a mechanical fastening.

A surgical robot R, not necessarily provided with haptic feedback, is considered to be available; it is not illustrated in the figures.

It is thus considered that the organ O can be reached by the surgeon C, and that access to the operating site and the related procedures of positioning the operating instruments have been completed.

The operation of the apparatus for detecting and tracking the position and/or deformation of a body organ subject to manipulation in an intraoperative context 1 according to the invention comprises the following steps.

### STEP_1

The devices D_1, ..., D_i, ..., D_N are initially arranged in a predefined position in order to be calibrated, i.e. so as to register the respective Cartesian reference systems with respect to the Cartesian reference system W.

Consequently, the transformations between a generic device D_i and the reference system W, and between W and the various D_1, ..., D_i, ..., D_N are known.

### STEP_2

The position of the display H is calibrated, i.e. the relevant Cartesian reference system, and this also means the one associated with the augmented reality space managed by H, is registered with respect to the Cartesian reference system W. Consequently, the transformations between H and W, and between W and H, and consequently the transformations between the devices D_1, ..., D_i, ..., D_N and H, and between H and the various devices D_1, ..., D_i, ..., D_N are known.

### STEP_3

The devices D_1, ..., D_i, ..., D_N are introduced into the abdomen and fixed to the surface of the organ O by the surgeon C, in such a way as to:
(i) prioritise the zones on the surface of O where he or she expects the maximum deformation induced by mechanical and manipulation stresses, and
(ii) if the acquisition of data by the devices D_1, ..., D_i, ..., D_N is via cable, maximise the probability that the cables will not obstruct the work of the surgeon C and in particular do not interfere with working space of the robot R.

### STEP_4

The devices D_1, ..., D_i, ..., D_N are powered, and then every device D_i generates a time series S_i at a certain frequency F_i.

The symbol S_i can also be understood as a reference to the specific inertial sensor of the associated device D_i.

It is realistically assumed that the frequencies are all equal, i.e. F_1 = ... = F_N, and thus that said frequencies can be referred to overall as F.

Every time series S_i is composed at every instant t of a pair (A_t, V_t), where
A_t indicates the linear acceleration vector and consists of a set of three (a_x, a_y, a_z), wherein a_x is the linear acceleration along the x axis, a_y is the linear acceleration along the y axis, a_z is the linear acceleration along the z axis (said axes are to be understood as with respect to the reference system of the device D_i, but transformable into the respective values with respect to the Cartesian reference system W), and
V_t indicates the angular velocity vector and consists of a set of three (v_x, v_y, v_z), wherein v_x is the angular velocity around the x axis, a_y is the angular velocity around the y axis, a_z is the angular velocity around the z axis (in this case as well, said axes are understood as with respect to the reference system of the device D_i, but transformable into the respective values with respect to the Cartesian reference system W).

S_it = (A_it, V_it) refers to the pair related to the time series S_i at the time instant t, for every i comprised between 1 and N and for every t between 1 and T, corresponding to the duration of the surgical procedure.

### STEP_5

For every device D_i and instant t, S_it is acquired by the sensor S_i, is transmitted by a transmitting unit (20), of which we now consider a specific instance defined as TX_i, is received by a receiving unit (30), of which we now consider a specific instance defined as RX, which concentrates the signals coming from all of the devices, and is then provided to an algorithm ALG_P which is run on a processing unit (40), of which we now consider a specific instance defined as a computing device E. This takes place at a frequency F.

### STEP_6

When the display H is worn by the surgeon C, the latter sees two superimposed representations of the world, the augmented space SA (generated artificially by H) and the common space SC.

In relation to the representation SA, the surgeon C sees the image I of the organ O floating in space and in a certain position PI.

Reference is made to that position in a certain instant t as PI_t, for every t comprised between 1 and T, corresponding to the duration of the surgical procedure.

At every instant t, PI_t is the result of the operations of the algorithm ALG_P, the behaviour of which is subsequently described in STEP 9.

In relation to the representation SC, the surgeon C sees the intraoperative context in which the position of O, to which we refer as PO, does not correspond to that of PI.

### STEP_7

Subsequently, the two positions PI and PO must be registered with respect to the reference system W.

This is done in an automatic mode.

An algorithm ALG_R implements a known technique of three-dimensional visual servoing.

ALG_R has as input the image I and the sensorial proximity data provided by H, conveniently represented as a vector RH of U elements such that RH = (Q_1, ..., Q_i, ..., Q_U), where every element Q_i is a set of three values (x_i, y_i, z_i) that represents the position, in the augmented space SA, of Q_i, with respect to the reference system of H, but it can obviously refer to W.

ALG_R has the purpose of superimposing I on O, and in particular of superimposing the two respective positions PI and PO.

ALG_R considers the subset RO of the vector RH, which contains the points Q_i in RH corresponding to the organ O, and implements a technique of minimising a cost function that depends on a distance metric between RI and RH in the augmented space SA, or a learning-based technique that determines an equivalent metric, or a technique based on multi-physical simulation.

The distance metric can advantageously be deterministic or probabilistic.

In the former case, when the value of the cost function is lower than a certain threshold SQ_1, it means that the image I and the organ O are superimposed, and in particular that the two positions PI and PO, respectively of the image and of the organ, are superimposed.

In the latter case, when the value of the cost function and of the main moments thereof are characterised by certain statistical properties defined as a whole as SQ_1, it means that the image I and the organ O are superimposed, and in particular that the two positions PI and PO, respectively of the image and of the organ, are superimposed.

### STEP_8

The surgeon C, during the operating step, manipulates the organ O by acting on the R robot, and consequently the organ O is moved.

From an operational viewpoint, it is advantageous to consider the organ O as a rigid body.

This implies that PI (respectively, PO) is a vector of values (p_x, p_y, p_z, o_x, o_y, o_z), where p_x is the position of I (respectively, O) relative to the x axis, p_y is the position of I (respectively, O) relative to the y axis, p_z is the position of I (respectively, O) relative to the z axis, o_x is the orientation of I (respectively, O) relative to the x axis, o_y is the orientation of I (respectively, O) relative to the y axis, o_z is the orientation of I (respectively, O) relative to the z axis, all with respect to the Cartesian reference system W.

### STEP_9

While the organ O is moved following the actions of the surgeon C, for every instant t, with t comprised between 1 and T, N data in the form of S_it = (A_it, V_it), one for every device D_i, are processed by ALG_P,

ALG_P implements a nonlinear probabilistic estimation algorithm, which can be obtained through learning models or be in a closed form; it generates the estimated values of the variables of PI_t using a model that integrates the values of A_it twice and the values of V_t once, thereby determining the position of the image I within the space SA registered with respect to the Cartesian reference system W.

### STEP_10

At every instant t, with t comprised between 1 and T, PI_t is used to update the position of the image I, and consequently the form of the vector RI within the space SA registered with respect to the Cartesian reference system W, as viewed by the surgeon C.

### STEP_11

As the surgeon C acts on the organ O, the various devices D_1, ..., D_i, ..., D_N, and for every instant of time t comprised between 1 and T, generate time series S_it which, processed by ALG_P, contribute to the calculation of the position PI_t of the image I and thus to the tracking of the position PO_t of the organ O through the estimation of the position PI_t.

From a technological viewpoint, the objective is to minimise, for every t comprised between 1 and T, a deterministic or probabilistic distance metric between PI_t and PO_t (which corresponds to the distance metric between RI and RH in the augmented space SA), and in any case to ensure that this is contained within (or is compatible with) the threshold SQ_1,

### STEP_12

When the surgical procedure is completed, that is, when t is equal to T, the devices D_1, ..., D_i, ..., D_N are removed from the surface of O.

A second preferred embodiment provides for an extension EXT-STEP_8 of STEP_8 described above, as follows.

The surgeon C, during the operating step, manipulates the organ O by acting on the robot R, and consequently the latter is deformed as a result of the surgical procedure.

From an operational viewpoint, it is now possible to consider the organ O as a deformable body.

It is assumed that the various devices D_1, ..., D_i, ..., D_N are positioned on the surface of O and that the various time series S_it = (A_it, V_it), with t comprised between 1 and T, represent the movements of the surface zones in which the various devices have been secured to O.

It is further assumed that a characterisation of the main mechanical characteristics of the organ O is available, for example in the form of stress-strain relations, or that there exists a model of such relations in multi-physical simulation, or in any case that said model can be obtained through learning techniques.

This implies that PI (respectively, PO) is a vector composed of N elements, each of the which in the form (p_x, p_y, p_z, o_x, o_y, o_z)_i, with i comprised between 1 and N, where p_x i-th is the position of D_i relative to the x axis, p_y i-th is the position of D_i relative to the y axis, p_z i-th is the position of D_i relative to the z axis, o_x i-th is the orientation of D_i relative to the x axis, o_y i-th is the orientation of D_i relative to the y axis and o_z i-th is the orientation of D_i relative to the z axis, all with respect to the Cartesian reference system W.

By means of known optimisation algorithms, it is possible to calculate or estimate the deformation of the image I of the organ O at the instant t DE_t on the basis of PI_t and the stress-strain relation of O, and consequently to determine I on the basis of PI_t and DE_t.

A third preferred embodiment comprises an alternative ALT-STEP_7 to STEP_7 as described above, as follows.

Following STEP_6, the two positions PI and PO must be registered with respect to the Cartesian reference system W.

This is done in an assisted mode by the surgeon C.

To begin with, an algorithm ALG RA executes the algorithm ALG_R, which implements a known three-dimensional visual servoing technique.

ALG_R has as input the image I and the sensorial proximity data provided by the device H, conveniently represented as a vector RH of U elements such that RH = (Q_1, ..., Q_i, ..., Q_U), where every element Q_i is a set of three values (x_i, y_i, z_i) that represents the position in the augmented space SA of Q_i, with respect to the reference system of H, but it can refer to W.

ALG_R has the purpose of superimposing I on O, and in particular of superimposing the two positions PI and PO.

ALG_R considers the subset RO of the vector RH, which contains the points Q_i in RH corresponding to the organ O, and implements a technique of minimising a cost function that depends on a distance metric between RI and RH in the augmented space SA, or a learning-based technique that determines an equivalent metric, or a technique based on multi-physical simulation.

The distance metric can advantageously be deterministic or probabilistic.

In the former case, when the value of the cost function is lower than a certain threshold SQ_1, it means that the image I and the organ O are superimposed, and in particular that the two positions PI and PO, respectively of the image and of the organ, are superimposed.

In the latter case, when the value of the cost function and of the main moments thereof are characterised by certain statistical properties defined as a whole as SQ_1, it means that the image I and the organ O are superimposed, and in particular that the two positions PI and PO, respectively of the image and of the organ, are superimposed.

In the event that the value of the cost function does not become lower than the threshold SQ_1 in the deterministic case, or the statistical properties of the value of the objective function are not compatible with those defined as SQ_1 in the probabilistic case within a certain time threshold SQ_2, the surgeon C is called on to make manual adjustments to PI, and then ALG_R resumes, starting from the data I associated with the PI obtained manually by the surgeon C.

These iterations are repeated in ALG_RA until the value of the cost function becomes lower than the threshold SQ_1 in the deterministic case, or the statistical properties become compatible with those of SQ_1, and it thus means that I and O are superimposed, and in particular that PI and PO are superimposed.

The manual adjustment can be made by the surgeon C in two steps:
(i) using a discrete movement by means of the interface of the device H, the surgeon C selects I, and
(ii) making a continuous movement, he or she carries out a rotation-translation of I in such a way as to superimpose I on O to the best of his or her ability.

It has in practice been observed that an apparatus for detecting and tracking the position and/or deformation of a body organ subject to manipulation in an intraoperative context, for example subject to surgical procedures according to the invention, is particularly advantageous for associating a three-dimensional augmented reality image of that organ with said representation of the position thereof and/or deformation thereof, in a simple, effective and safe manner.

Another advantage of the invention is to provide an apparatus for detecting and tracking the position and deformation of a body organ in an intraoperative context, for example during surgical procedures, which enables assisted intracorporeal orientation to be achieved in the course of surgical procedures in the intraoperative context by overlaying a three-dimensional augmented reality image with the position and/or deformation of the body organ in the common three-dimensional space through a wearable or portable display device with a valid, effective and certifiable real-time synchrony with the visual field of medical staff.

Another advantage of the invention is that of providing an apparatus for detecting and tracking the position and/or deformation of a body organ in an intraoperative context, for example in the course of surgical procedures, which updates the position and/or deformation of the three-dimensional augmented reality image in correspondence with the position and/or deformation of the body organ, and of providing data of an absolute or time-differential type and localised on the surface of that organ.

An apparatus for detecting and tracking the position and/or deformation of a body organ subject to manipulation in an intraoperative context, for example subject a surgical procedures, thus conceived is susceptible of numerous modifications and variants, all falling within the scope of the inventive concept, as defined by the claims.

In practice, the materials and the devices used, as well as the dimensions, parameters and algorithms, can be any whatsoever according to needs and the state of science and technology.

## Claims

1. A detection and tracking apparatus (1) for detecting and tracking at least the position of a body organ subject to manipulation, said body organ being selected among thoracic internal organs, abdominal organs and pelvic organs, having:
- at least one detection sensor (11) for detecting the position of said body organ (10), configured to provide information of an absolute or time-differential type;
- a rigid coupling means (12) for coupling said at least one detection sensor (11) to said body organ (10);
- at least one transmitting unit (20) for transmitting the information about said position of an absolute or time-differential type detected by said detection sensor (11);
- at least one receiving unit (30) for receiving said information about said position of an absolute or time-differential type transmitted by said transmitting unit (20);
**characterised in that** it further has:
- at least one wearable and/or portable display device (50);
- at least one processing unit (40), in communication with said receiving unit (30) and with said display device (50), configured to perform the following steps of a procedure for evaluating said information about said position of an absolute or time-differential type:
a) determining a representation of said position of said organ (10), said processing unit (40) having an inductive or deductive algorithm for determining said representation;
b) associating a three-dimensional augmented reality image (100) of said organ (10) with said representation of said position thereof;
c) overlaying said three-dimensional augmented reality image (100) with the position of said organ (10) in a common three-dimensional space through said wearable and/or portable display device (50);
d) tracking position of said three-dimensional augmented reality image (100) by superimposing it with the position of said organ (10) of which said three-dimensional augmented reality image (100) is a representation, comparing said information about said position with a plurality of predefined models of position of organs.

2. A detection and tracking apparatus (1) according to claim 1, **characterised in that** it has at least two detection sensors (11) for detecting the position and deformation of said body organ (10); said at least one transmitting unit (20) being configured to transmit the information about said position and said deformation of an absolute or time-differential type detected by said detection sensors (11); said at least one receiving unit (30) being configured to receive said information about said position and said deformation of an absolute or time-differential type transmitted by said transmitting unit (20); said at least one processing unit (40) being configured to determine a representation of said position and said deformation of said organ (10), associate a three-dimensional augmented reality image (100) of said organ (10) with said representation of said position and said deformation thereof, overlaying said three-dimensional augmented reality image (100) with the position and deformation of said organ (10) in the common three-dimensional space through said wearable and/or portable display device (50); tracking said position and said deformation of said three-dimensional augmented reality image (100) in correspondence with the position and deformation of said organ (10) of which said three-dimensional augmented reality image (100) is a representation, comparing said information about said position and said deformation with a plurality of predefined models of position and deformation of organs.

3. The detection and tracking apparatus (1) according to any previous claim, **characterised in that** said at least one detection sensor (11) is an inertial sensor.

4. The detection and tracking apparatus (1) according to one or more of the preceding claims, **characterised in that** said coupling means (12) for coupling said at least one detection sensor (11) to said body organ (10) are mechanical fixing means.

5. The detection and tracking apparatus (1) according to one or more of the preceding claims, **characterised in that** said at least one wearable and/or portable display device (50) is a see-through augmented reality display.

6. The detection and tracking apparatus (1) according to any previous claim, **characterised in that** said inductive or deductive algorithm for determining said representation comprises a nonlinear probabilistic estimation algorithm for estimating the position and/or deformation of said body organ which generates estimated values using a model that integrates acceleration values two times and velocity values one time.

7. The detection and tracking apparatus (1) according to the preceding claim, **characterised in that** said estimation algorithm is obtained by means of learning models.

8. The detection and tracking apparatus (1) according to claim 6, **characterised in that** said estimation algorithm is in a closed form.

9. The detection and tracking apparatus (1) according to any previous claim, **characterised in that** said at least one detection sensor (11) comprises an accelerometer or a gyroscope or magnetometer configured for the transmission of data via a cable or by means of a wireless connection.

10. The detection and tracking apparatus (1) according to any previous claim, **characterised in that** said at least one processing unit (40) also performs a learning step based on algorithms and procedures of an inductive type whereby, for every type of body organ, a corresponding model for determining the position and/or deformation is generalised.

## Patentansprüche

1. Detektions- und Ortungsvorrichtung (1) zum Detektieren und Orten mindestens der Position eines zu manipulierenden Körperorgans, wobei das Körperorgan aus inneren Organen des Brustraums, Organen des Bauchraums und Organen des Beckenraums ausgewählt ist, umfassend:
- mindestens einem Detektionssensor (11) zum Detektieren der Position des Körperorgans (10), der ausgebildet ist, Informationen eines absoluten oder zeitdifferenziellen Typs zu liefern;
- starre Kopplungsmittel (12) zum Koppeln des mindestens einen Detektionssensors (11) mit dem Körperorgan (10);
- mindestens eine Sendeeinheit (20) zum Übertragen der von dem Detektionssensor (11) detektierten Informationen eines absoluten oder zeitdifferenziellen Typs über die Position;
- mindestens eine Empfangseinheit (30) zum Empfangen der von der Sendeeinheit (20) übertragenen Informationen eines absoluten oder zeitdifferenziellen Typs über die Position;
**dadurch gekennzeichnet, dass** sie ferner umfasst:
- mindestens eine tragbare und/oder portable Anzeigevorrichtung (50);
- mindestens eine Verarbeitungseinheit (40), die mit der Empfangseinheit (30) und mit der Anzeigevorrichtung (50) in Verbindung steht und ausgebildet ist, die folgenden Schritte eines Verfahrens zum Auswerten der Informationen eines absoluten oder zeitdifferenziellen Typs über die Position auszuführen:
a) Bestimmen einer Darstellung der Position des Organs (10), wobei die Verarbeitungseinheit (40) einen induktiven oder deduktiven Algorithmus zum Bestimmen der Darstellung aufweist;
b) Zuordnen eines dreidimensionalen Augmented-Reality-Bildes (100) des Organs (10) zu der Darstellung seiner Position;
c) Überlagern des dreidimensionalen Augmented-Reality-Bildes (100) mit der Position des Organs (10) in einem gemeinsamen dreidimensionalen Raum durch die tragbare und/oder portable Anzeigevorrichtung (50);
d) Orten der Position des dreidimensionalen Augmented-Reality-Bildes (100), durch Überlagern desselben mit der Position des Organs (10), dessen Darstellung das dreidimensionale Augmented-Reality-Bild (100) ist, Vergleichen der Informationen über die Position mit einer Vielzahl an vorgegebenen Modellen von Positionen von Organen.

2. Detektions- und Ortungsvorrichtung (1) nach Anspruch 1, **dadurch gekennzeichnet, dass** sie mindestens zwei Detektionssensoren (11) zum Detektieren der Position und Verformung des Körperorgans (10) aufweist; wobei die mindestens eine Sendeeinheit (20) ausgebildet ist, die Informationen eines absoluten oder zeitdifferenziellen Typs über die Position und die Verformung, die von den Detektionssensoren (11) detektiert wurden, zu übertragen; wobei die mindestens eine Empfangseinheit (30) ausgebildet ist, die von der Sendeeinheit (20) übertragenen Informationen eines absoluten oder zeitdifferenziellen Typs über die Position und die Verformung zu empfangen; wobei die mindestens eine Verarbeitungseinheit (40) ausgebildet ist, eine Darstellung der Position und der Verformung des Organs (10) zu bestimmen, einem dreidimensionalen Augmented-Reality-Bild (100) des Organs (10) die Darstellung der Position und der Verformung desselben zuzuordnen, das dreidimensionale Augmented-Reality-Bild (100) mit der Position und der Verformung des Organs (10) in dem gemeinsamen dreidimensionalen Raum durch die tragbare und/oder portable Anzeigevorrichtung (50) zu überlagern, die Position und die Verformung des dreidimensionalen Augmented-Reality-Bildes (100) in Übereinstimmung mit der Position und der Verformung des Organs (10), von dem das dreidimensionale Augmented-Reality-Bild (100) eine Darstellung ist, zu orten, die Informationen über die Position und die Verformung mit einer Vielzahl vorgegebener Modelle von Positionen und Verformungen von Organen zu vergleichen.

3. Detektions- und Ortungsvorrichtung (1) nach einem der vorhergehenden Ansprüche, **dadurch gekennzeichnet, dass** der mindestens eine Detektionssensor (11) ein Trägheitssensor ist.

4. Detektions- und Ortungsvorrichtung (1) nach einem oder mehreren der vorhergehenden Ansprüche, **dadurch gekennzeichnet, dass** die Kopplungsmittel (12) zum Koppeln des mindestens einen Detektionssensors (11) mit dem Körperorgan (10) mechanische Befestigungsmittel sind.

5. Detektions- und Ortungsvorrichtung (1) nach einem oder mehreren der vorhergehenden Ansprüche, **dadurch gekennzeichnet, dass** die mindestens eine tragbare und/oder portable Anzeigevorrichtung (50) eine durchsichtige Augmented-Reality-Anzeige ist.

6. Detektions- und Ortungsvorrichtung (1) nach einem der vorhergehenden Ansprüche, **dadurch gekennzeichnet, dass** der induktive oder deduktive Algorithmus zum Bestimmen der Darstellung einen nichtlinearen wahrscheinlichkeitsbasierten Schätzalgorithmus zum Schätzen der Position und/oder Verformung des Körperorgans umfasst, der geschätzte Werte unter Verwendung eines Modells erzeugt, das Beschleunigungswerte zweimal und Geschwindigkeitswerte einmal integriert.

7. Detektions- und Ortungsvorrichtung (1) nach dem vorhergehenden Anspruch, **dadurch gekennzeichnet, dass** der Schätzalgorithmus mittels Lernmodellen erhalten wird.

8. Detektions- und Ortungsvorrichtung (1) nach Anspruch 6, **dadurch gekennzeichnet, dass** der Schätzalgorithmus in einer geschlossenen Form vorliegt.

9. Detektions- und Ortungsvorrichtung (1) nach einem der vorhergehenden Ansprüche, **dadurch gekennzeichnet, dass** der mindestens eine Detektionssensor (11) einen Beschleunigungsmesser oder ein Gyroskop oder Magnetometer umfasst, der bzw. das zur Übertragung von Daten über ein Kabel oder mittels einer drahtlosen Verbindung ausgebildet ist.

10. Detektions- und Ortungsvorrichtung (1) nach einem der vorhergehenden Ansprüche, **dadurch gekennzeichnet, dass** die mindestens eine Verarbeitungseinheit (40) auch einen Lernschritt auf der Grundlage von Algorithmen und Verfahren induktiver Art durchführt, wobei für jeden Typ von Körperorgan ein entsprechendes Modell zur Bestimmung der Position und/oder Verformung verallgemeinert wird.

## Revendications

1. - Appareil de détection et de suivi (1) pour détecter et suivre au moins la position d'un organe corporel soumis à une manipulation, ledit organe corporel étant choisi parmi des organes internes thoraciques, des organes abdominaux et des organes pelviens, ayant :
- au moins un capteur de détection (11) pour détecter la position dudit organe corporel (10), configuré pour fournir des informations d'un type différentiel temporel ou absolu ;
- un moyen de couplage rigide (12) pour coupler ledit au moins un capteur de détection (11) audit organe corporel (10) ;
- au moins une unité de transmission (20) pour transmettre les informations sur ladite position d'un type différentiel temporel ou absolu détectées par ledit capteur de détection (11) ;
- au moins une unité de réception (30) pour recevoir lesdites informations sur ladite position d'un type différentiel temporel ou absolu transmises par ladite unité de transmission (20) ;
**caractérisé par le fait qu'**il a en outre :
- au moins un dispositif d'affichage à porter sur soi et/ou portable (50) ;
- au moins une unité de traitement (40), en communication avec ladite unité de réception (30) et avec ledit dispositif d'affichage (50), configurée pour réaliser les étapes suivantes d'une procédure d'évaluation desdites informations sur ladite position d'un type différentiel temporel ou absolu :
a) déterminer une représentation de ladite position dudit organe (10), ladite unité de traitement (40) ayant un algorithme inductif ou déductif pour déterminer ladite représentation ;
b) associer une image tridimensionnelle de réalité augmentée (100) dudit organe (10) à ladite représentation de ladite position de celui-ci ;
c) superposer ladite image tridimensionnelle de réalité augmentée (100) avec la position dudit organe (10) dans un espace tridimensionnel commun par l'intermédiaire dudit dispositif d'affichage à porter sur soi et/ou portable (50) ;
d) suivre la position de ladite image tridimensionnelle de réalité augmentée (100) par superposition de celle-ci avec la position dudit organe (10) dont ladite image tridimensionnelle de réalité augmentée (100) est une représentation, comparer lesdites informations sur ladite position à une pluralité de modèles prédéfinis de position d'organes.

2. - Appareil de détection et de suivi (1) selon la revendication 1, **caractérisé par le fait qu'**il comporte au moins deux capteurs de détection (11) pour détecter la position et la déformation dudit organe corporel (10) ; ladite au moins une unité de transmission (20) étant configurée pour transmettre les informations sur ladite position et ladite déformation d'un type différentiel temporel ou absolu détectées par lesdits capteurs de détection (11) ; ladite au moins une unité de réception (30) étant configurée pour recevoir lesdites informations sur ladite position et ladite déformation d'un type différentiel temporel ou absolu transmises par ladite unité de transmission (20) ; ladite au moins une unité de traitement (40) étant configurée pour déterminer une représentation de ladite position et de ladite déformation dudit organe (10), associer une image tridimensionnelle de réalité augmentée (100) dudit organe (10) à ladite représentation de ladite position et de ladite déformation de celui-ci, superposer ladite image tridimensionnelle de réalité augmentée (100) à la position et à la déformation dudit organe (10) dans l'espace tridimensionnel commun par l'intermédiaire dudit dispositif d'affichage à porter sur soi et/ou portable (50) ; suivre ladite position et ladite déformation de ladite image tridimensionnelle de réalité augmentée (100) en correspondance avec la position et la déformation dudit organe (10) dont ladite image tridimensionnelle de réalité augmentée (100) est une représentation, comparer lesdites informations sur ladite position et ladite déformation à une pluralité de modèles prédéfinis de position et de déformation d'organes.

3. - Appareil de détection et de suivi (1) selon l'une quelconque des revendications précédentes, **caractérisé par le fait que** ledit au moins un capteur de détection (11) est un capteur inertiel.

4. - Appareil de détection et de suivi (1) selon une ou plusieurs des revendications précédentes, **caractérisé par le fait que** lesdits moyens de couplage (12) pour coupler ledit au moins un capteur de détection (11) audit organe corporel (10) sont des moyens de fixation mécanique.

5. - Appareil de détection et de suivi (1) selon une ou plusieurs des revendications précédentes, **caractérisé par le fait que** ledit au moins un dispositif d'affichage à porter sur soi et/ou portable (50) est un dispositif d'affichage de réalité augmentée transparent.

6. - Appareil de détection et de suivi (1) selon l'une quelconque des revendications précédentes, **caractérisé par le fait que** ledit algorithme inductif ou déductif pour déterminer ladite représentation comprend un algorithme d'estimation probabiliste non linéaire pour estimer la position et/ou la déformation dudit organe corporel qui génère des valeurs estimées à l'aide d'un modèle qui intègre des valeurs d'accélération à deux instants et des valeurs de vitesse à un instant.

7. - Appareil de détection et de suivi (1) selon la revendication précédente, **caractérisé par le fait que** ledit algorithme d'estimation est obtenu au moyen de modèles d'apprentissage.

8. - Appareil de détection et de suivi (1) selon la revendication 6, **caractérisé par le fait que** ledit algorithme d'estimation est dans une forme fermée.

9. - Appareil de détection et de suivi (1) selon l'une quelconque des revendications précédentes, **caractérisé par le fait que** ledit au moins un capteur de détection (11) comprend un accéléromètre ou un gyroscope ou un magnétomètre configuré pour la transmission de données par l'intermédiaire d'un câble ou au moyen d'une connexion sans fil.

10. - Appareil de détection et de suivi (1) selon l'une quelconque des revendications précédentes, **caractérisé par le fait que** ladite au moins une unité de traitement (40) effectue également une étape d'apprentissage basée sur des algorithmes et des procédures d'un type inductif permettant de généraliser, pour chaque type d'organe corporel, un modèle correspondant de détermination de la position et/ou de la déformation.
